# EUROPÄISCHE PATENTSCHRIFT

(11) **EP 1 387 659 B1**
(45) Veröffentlichungstag und Bekanntmachung des Hinweises auf die Patenterteilung: **07.05.2008**
(21) Anmeldenummer: 02753050.0
(22) Anmeldetag: 13.05.2002
(51) Int. Cl.: A61K 6/083

(54) **INITIATORSYSTEM FÜR SAURE DENTALFORMULIERUNGEN**
INITIATOR SYSTEM FOR ACID DENTAL FORMULATIONS
SYSTEME INITIATEUR POUR FORMULATIONS DENTAIRES ACIDES

(30) Priorität: 16.05.2001 DE 10124029
(43) Veröffentlichungstag der Anmeldung: 11.02.2004
(73) Patentinhaber: 3M ESPE AG, 82229 Seefeld (DE)
(72) Erfinder: HECHT, Reinhold, 86916 Kaufering (DE); LUDSTECK, Manfred, 82538 Geretsried (DE)
(86) Internationale Anmeldenummer: PCT/EP2002/005219
(87) Internationale Veröffentlichungsnummer: WO 2002/092023

(56) Entgegenhaltungen:
- EP-A- 0 374 824
- EP-A- 0 923 924
- DE-A- 4 219 700
- DE-A- 19 757 277
- DE-A- 19 928 238
- "Römpp - Chemielexikon" 1995, GEROG THIEME VERLAG * Seiten 892,3811 *

## Beschreibung

Die vorliegende Erfindung betrifft ein Redox-Initiatorsystem, welches die Aushärtung dentaler Formulierungen im sauren Milieu über eine radikalische Polymerisation ermöglicht und für eine hohe Haftung der polymerisierenden Massen an den Zahnhartsubstanzen Schmelz und insbesondere Dentin sorgt.

Materialien auf Basis von Acrylaten und Methacrylaten sind für viele zahnärztliche Anwendungen die Materialien der Wahl und als wesentliche Bestandteile der Formulierungen von Bondings und Compositen sowie den Compomeren und kunststoffverstärkten Glasionomerzementen bekannt. Die Aushärtung dieser Systeme erfolgt üblicherweise über eine radikalische Polymerisation, wobei die Radikale über geeignete Initiatorsysteme photochemisch, thermisch und/oder durch Redoxreaktionen zur Verfügung gestellt werden. Das Polymerisationsinitiatorsystem hat dabei einen entscheidenden Einfluß auf die Qualität des Dentalmaterials.

Es ist Stand der Technik, (meth)acrylathaltige Dentalmaterialien photochemisch zu härten. Insbesondere der Einsatz von Campherchinon in Kombination mit tertiären Aminen ist in zahlreichen Veröffentlichungen wie E. Andrzejewska et al., Macromol. Chem. Phys. 199, 441-449 (1998) und EP 0132 959 A1 oder EP 0047097 B1 beschrieben.

Eine weitere Möglichkeit eine radikalische Polymerisation zu starten besteht im thermischen Zerfall geeigneter Ausgangsmoleküte. So werden in der Zahntechnik für sogenannte Heißpolymerisate Peroxide wie Benzoylperoxid, Lauroylperoxid oder tert.-Butylperbenzoat sowie weitere, beispielsweise in der EP 0410199 A beschriebene Verbindungen, eingesetzt.

Der Einsatz thermisch arbeitender Initiatorsysteme ist in der Regel auf zahntechnische Produkte limitiert, da die erforderlichen Temperaturen für zahnmedizinische intraorale Anwendungen nicht akzeptabel sind. Auch für den Einsatz von Photoinitiatoren gibt es limitierende Faktoren. Beispielsweise ist bei der Zementierung von Metallrestaurationen mit Zementen auf Composit-Basis eine Lichtinitiierung nicht möglich. Ferner können mit Licht nicht beliebig dicke Schichten ausgehärtet werden, d.h. bei der Füllung von tiefen Kavitäten ist eine Bulkfüllung nicht möglich und es muß in der sogenannten Schichttechnik gearbeitet werden (siehe W. Geurtsen, Klinik der Kompositfüllung, Hanser Verlag München, Wien, 1989).

Zur Lösung der genannten Probleme wurden im Dentalbereich sog. Redoxinitiatorsysteme vorgeschlagen. Es handelt sich hierbei um eine Kombination eines Reduktions- mit einem Oxidationsmittel, wobei die Bestandteile des Redoxsystems aus Stabilitätsgründen getrennt gelagert werden. Es resultieren daher Paste / Paste-, Pulver / Flüssigkeits- oder Flüssigkeit/Flüssigkeitssysteme. Nach dem Anmischen dieser Systeme werden durch das Redoxsystem Radikale bereits bei niedrigen Temperaturen gebildet.

Bekannte Redoxsysteme sind z.B. Kombinationen aus organischen Peroxiden wie Benzoyl- oder Lauroylperoxid mit tertiären aromatischen Aminen wie N,N-Dimethyl-p-toluidin oder anderen strukturverwandten Aminen wie sie z.B. in der US 3,541,068 oder DE 26 58 538 A beschrieben werden. Nachteilig an den Peroxid / Aminsystemen ist deren fehlende Fähigkeit saure Harzsysteme, wie sie im Dentalbereich beispielsweise bei Bondings vorliegen, optimal auszuhärten.

Weitere bekannte Redoxsysteme basieren auf Derivaten der Barbitur- bzw. Thiobarbitursäure. In der EP 0 480 785 A werden Thiobarbitursäure- oder Barbitursäureverbindungen in Kombination mit Kupfer- bzw. Eisenhalogeniden als Polymerisationsinitiatoren für die radikalische Polymerisation offenbart.

Die DE 42 19 700 A beschreibt ein Initiatorsystem, bestehend aus einem Thiobarbitursäurederivat, Kupfersalz und Chloridionen zur Aushärtung von 2-Hydroxyethylmethacrylat. In B. Bredereck et al., Makromol. Chem. 92, 70 (1966) und der DE 14 95 520 A werden Kombinationen von Barbitur- und Thiobarbitursäurederivaten mit Peroxiden, Kupferverbindungen und Chloridionen (sog. Bredereck-System) beschrieben. Alle genannten Systeme bewirken im sauren Milieu nur eine unzureichende Aushärtung der Massen, so dass hohe Löslichkeiten, niedrige mechanische Werte und geringe Farbstabilitäten resultieren.

Die US 5,688,883 beschreibt ein System, bestehend aus einem organischen Peroxid mit einer Halbwertszeit von 10 Stunden bei Temperaturen von über 75°C, einem Protonendonor (z.B. einem Barbitursäurederivat) und einer Metallverbindung (z.B. einer Kupferverbindung). Dieses System ist in der Lage auch saure Massen zu polymerisieren. Allerdings bewirkt dieses System keine Haftung zwischen dem polymerisierenden Material und der Zahnhartsubstanz.

Die DE 19 757 277 A1 offenbart ein Initiatorsystem, bestehend aus einem Kupfersalz, einer Sulfinsäureverbindung und einem Barbitur- bzw. Thiobarbitursäurederivat. Durch dieses System lassen sich saure Zahnharzsysteme aushärten und es wird eine Haftung an der Zahnhartsubstanz erreicht. Die Haftwerte bewegen sich allerdings auf einem vergleichsweise niedrigem Niveau.

Aus den vorher genannten Gründen wird ersichtlich, dass im Dentalbereich erheblicher Bedarf an einem Redox-Initiatorsystem besteht, welches eine effektive Aushärtung gefüllter und ungefüllter Formulierungen, vorzugsweise im sauren Milieu ermöglicht und zu hohen Haftwerten an Dentin und Schmelz beiträgt.

Überraschenderweise wurde gefunden, dass sich die erfindungsgemäße Aufgabe durch ein Redox-Initiatorsystem, welches folgende Bestandteile umfasst, lösen lässt:
(A) 14,9 bis 50 Gew.-%, bevorzugt 20 bis 45 Gew.-% einer Barbitursäure oder Thiobarbitursäure bzw. eines Barbitursäure- oder Thiobarbitursäurederivats,
(B) 30 bis 75 Gew.-%, bevorzugt 35 bis 67,8 Gew.-% einer Peroxodisulfatverbindung und/oder Peroxodiphosphatverbindung.
(C) 10 bis 35 Gew.-%, bevorzugt 12 bis 30 Gew.-% einer Sulfinsäureverbindung und
(D) 0,1 bis 5 Gew.-%, bevorzugt 0,2 bis 4 Gew.-% einer Kupferverbindung.

Mit den Begriffen "umfassen", "aufweisen" oder "enthalten" wird eine nicht abschließende Aufzählung von Merkmalen eingeleitet. Gleichermaßen ist der Begriff "ein" im Sinne von "mindestens eins" zu verstehen.

Bei der Komponente (A) handelt es sich um eine Barbitursäure oder Thiobarbitursäure bzw. ein Barbitursäure- oder Thiobarbitursäurederivat bzw. Mischungen davon der allgemeinen Struktur:

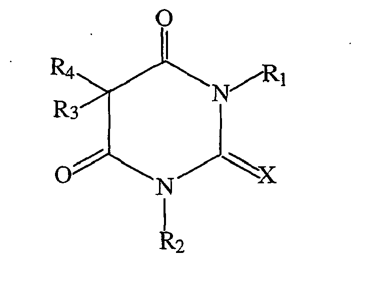

Dabei bedeutet X = O oder S. R₁, R₂, R₃ und R₄, die gleich oder verschieden sein können, bedeuten: Wasserstoff, Alkyl-, substituierter Alkyl-, Alkenyl-, Cycloalkyl-, substituierter Cycloalkyl-, Arylalkyl-, Aryl- oder substituierter Arylrest. R₁, R₂, R₃ und R₄ können ferner einen Halogenrest wie Chlor oder eine Hydroxy-, Amino- oder Nitrogruppe verkörpern.

Wenn einer der Reste R₁ bis R₄ unsubstituiertes Alkyl bedeutet, so kann dieser Rest gerade oder verzweigt sein und beispielsweise 1 bis 18 Kohlenstoffatome, vorzugsweise 1 bis 10 und insbesondere 1 bis 6 Kohlenstoffatome aufweisen. Beispiele für niedrigmolekulare Alkylreste sind Methyl, Ethyl, Propyl, i-Propyl, n-Butyl, t-Butyl, i-Butyl, n-Pentyl und Isoamyl.

Wenn einer der Reste R₁ bis R₄ einen substituierten Alkylrest darstellt, so weist der Alkylteil dieses Restes vorzugsweise die Anzahl von Kohlenstoffatomen auf, die oben für unsubstituiertes Alkyl angegeben sind. Ist einer der Reste R₁ bis R₄ Alkoxyalkyl oder Alkoxycarbonylalkyl, so enthält der Alkoxyrest beispielsweise 1 bis 5 Kohlenstoffatome und ist vorzugsweise Methyl, Ethyl, Propyl, i-Propyl, n-Butyl, t-Butyl, i-Butyl, n-Pentyl oder Isoamyl. Ist einer der Reste R₁ bis R₄ Halogenalkyl, so wird unter Halogen Fluor, Chlor, Brom oder Jod verstanden.

Bedeutet einer der Reste R₁ bis R₄ Alkenyl, so sind C₃ bis C₅ -Alkenylreste, insbesondere Allyl, bevorzugt.

Wenn einer der Reste R₁ bis R₄ unsubstituiertes Cycloalkyl bedeutet, so sind C₄ bis C₇ -Cycloalkylreste bevorzugt. Besonders bevorzugt sind Cyclopentyl und Cyclohexyl.

Bedeutet einer der Reste R₁ bis R₄ substituiertes Cycloalkyl, so sind die vorstehend angegebenen Cycloalkylreste bevorzugt, wobei der oder die Substituenten am Cycloalkylrest z.B. C₁ bis C₄-Alkyl wie Methyl, Ethyl, Propyl, n-Butyl oder i-Butyl, Fluor, Chlor, Brom, Jod oder C₁ bis C₄ -Alkoxy, insbesondere Methoxy sein können.

Wenn einer der Reste R₁ bis R₄ Aryl oder Aralkyl bedeutet, so sind Phenyl und Naphthyl als Aryl bevorzugt. Besonders bevorzugte Arylalkylreste sind Benzyl und Phenylethyl.

R₁ bis R₄ können gegebenenfalls auch substituierte Arylreste sein. Hier sind Phenyl und Naphthyl bevorzugt und als Ringsubstituenten C₁ bis C₄ -Alkyl, besonders Methyl, Halogen oder C₁ bis C₄-Alkoxy, besonders Methoxy.

Als Vertreter der Komponente (A) seien exemplarisch genannt: Barbitursäure, Thiobarbitursäure, 1,3,5-Trimethylbarbitursäure, 1-Phenyl-5-benzyl-barbitursäure, 1-Benzyl-5-phenyl-barbitursäure, 1,3-Dimethylbarbitursäure, 1,3-Dimethyl-5-phenyl-barbitursäure, 1-Cyclohexyl-5-ethyl-barbitursäure, 5-Laurylbarbitursäure, 5-Butylbarbitursäure, 5-Allylbarbitursäure, 5-Hydroxy-5-butylbarbitursäure, 5-Phenylthiobarbitursäure, 1,3-Dimethylthiobarbitursäure, 5,5-Dibrombarbitursäure, Trichlorbarbitursäure, 5-Nitrobarbitursäure, 5-Aminobarbitursäure, 5-Hydroxybarbitursäure und 5,5-Dihydroxybarbitursäure.

Die Peroxodisulfatverbindungen und/oder Peroxodiphosphatverbindung der Komponente (B) bzw. Mischungen davon können organischer bzw. anorganischer Natur sein, wobei salzartige Verbindungen, insbesondere wasserlösliche Verbindungen bevorzugt sind. Wasserlöslich in diesem Zusammenhang heißt, eine Löslichkeit von mindestens 4g Substanz / 100 ml Wasser bei 20°C, vorzugsweise von mindestens 10 g Substanz / 100 ml Wasser bei 20°C, besonders bevorzugt von mindestens 20 g Substanz / 100 ml Wasser bei 20°C.

Exemplarisch seien genannt die Ammonium-, Natrium- und Kaliumperoxodisulfatverbindungen bzw. -peroxodiphosphatverbindugnen. Besonders bevorzugt ist Natriumperoxodisulfat.

Es hat sich gezeigt, daß andere Peroxoverbindungen, insbesondere organische Peroxoverbindungen zu keinen brauchbaren Resultaten führen.

Bei der Komponente (C) handelt es sich um eine Sulfinsäureverbindung bzw. Mischungen davon der allgemeinen Formel R₁SOO-R₂, wobei R₁ ein Alkyl-, substituierter Alkyl-, Alkenyl-, Cycloalkyl-, substituierter Cycloalkyl-, Arylalkyl-, Aryl- oder substituierter Arylrest ist und R₂ = H, Metall wie z.B. Lithium, Natrium oder Kalium oder ein Alkyl-, substituierter Alkyl-, Alkenyl-, Cycloalkyl-, substituierter Cycloalkyl-, Arylalkyl-, Aryl- oder substituierter Arylrest ist.

Wenn einer der Reste R₁ oder R₂ unsubstituiertes Alkyl bedeutet, so kann dieser Rest gerade oder verzweigt sein und beispielsweise 1 bis 18 Kohlenstoffatome, vorzugsweise 1 bis 10 und insbesondere 1 bis 6 Kohlenstoffatome aufweisen. Beispiele für niedrigmolekulare Alkylreste sind Methyl, Ethyl, Propyl, i-Propyl, n-Butyl, t-Butyl, i-Butyl, n-Pentyl und Isoamyl.

Wenn einer der Reste R₁ oder R₂ einen substituierten Alkylrest darstellt, so weist der Alkylteil dieses Restes vorzugsweise die Anzahl von Kohlenstoffatomen auf, die oben für unsubstituiertes Alkyl angegeben sind. Ist einer der Reste R₁ oder R₂ Alkoxyalkyl oder Alkoxycarbonylalkyl, so enthält der Alkoxyrest beispielsweise 1 bis 5 Kohlenstoffatome und ist vorzugsweise Methyl, Ethyl, Propyl, i-Propyl, n-Butyl, t-Butyl, i-Butyl, n-Pentyl oder Isoamyl. Ist einer der Reste R₁ oder R₂ Halogenalkyl, so wird unter Halogen Fluor, Chlor, Brom oder Jod verstanden.

Bedeutet einer der Reste R₁ oder R₂ Alkenyl, so sind C₃ bis C₅ -Alkenylreste, insbesondere Allyl, bevorzugt.

Wenn einer der Reste R₁ oder R₂ unsubstituiertes Cycloalkyl bedeutet, so sind C₄ bis C₇ -Cycloalkylreste bevorzugt. Besonders bevorzugt sind Cyclopentyl und Cyclohexyl.

Bedeutet einer der Reste R₁ oder R₂ substituiertes Cycloalkyl, so sind die vorstehend angegebenen Cycloalkylreste bevorzugt, wobei der oder die Substituenten am Cycloalkylrest z.B. C₁ bis C₄-Alkyl wie Methyl, Ethyl, Propyl, n-Butyl oder i-Butyl, Fluor, Chlor, Brom, Jod oder C₁ bis C₄-Alkoxy, insbesondere Methoxy sein können.

Wenn einer der Reste R₁ oder R₂ Aryl oder Aralkyl bedeutet, so sind Phenyl und Naphthyl als Aryl bevorzugt. Besonders bevorzugte Arylalkylreste sind Benzyl und Phenylethyl.

R₁ oder R₂ können gegebenenfalls auch substituierte Arylreste sein. Hier sind Phenyl und Naphthyl bevorzugt und als Ringsubstituenten C₁ bis C₄ -Alkyl, besonders Methyl, Halogen oder C₁ bis C₄-Alkoxy, besonders Methoxy.

Exemplarisch als Vertreter der Komponente (C) seien genannt: Benzolsulfinsäure, Natriumbenzolsulfinat, Natriumbenzolsulfinatdihydrat, Natriumtoluolsulfinat, Formamidinsulfinsäure, Natriumsalz der Hydroxymethansulfinsäure, Natriumsalz der 2,5-Dichlorbenzolsulfinsäure, 3-Acetamido-4-methoxybenzolsulfinsäure.

Besonders bevorzugte Verbindungen der Komponente (C) sind Natriumtoluolsulfinat oder Natriumbenzolsulfinat bzw. deren Hydrate.

Die Kupferverbindung (D) bzw. Mischungen davon besitzen die allgemeine Formel CuXₙ, wobei X ein anorganisches und/oder organisches Anion ist und n = 1 oder 2 ist. Geeignete Kupferverbindungen sind z.B. Kupferchlorid, Kupferacetat, Kupferacetylacetonat, Kupfernaphthenat, Kupfersalicylat oder Komplexe von Kupfer mit Thioharnstoff oder Ethylendiamintetraessigsäure. Besonders bevorzugt ist Kupferacetat.

Der Anteil des erfindungsgemäßen Initiatorsystems, umfassend die Komponenten (A) bis (D), in den dentalen Formulierungen beträgt 0,5 bis 15 Gew.% und besonders bevorzugt 1,0 bis 10 Gew.%. Bei den dentalen Formulierungen handelt es sich um Pulver/Flüssigkeits-, Paste/Paste- bzw. Flüssigkeits/Flüssigkeitssysteme. Besonders bevorzugt sind Pulver/Flüssigkeits-Systeme. Aus Gründen der Lagerstabilität können die Bestandteile des erfindungsgemäßen Initiatorsystems mikroverkapselt werden. Verfahren zur Mikroverkapselung sind beispielsweise in der US 5 154 762 und EP 0 588 878 B1 beschrieben.

Die dentalen Formulierungen weisen unmittelbar nach dem Mischen einen pH-Wert von kleiner 5, bevorzugt von kleiner 3 und ganz besonders bevorzugt von kleiner 1 auf. Der pH-Wert wurde gemäß der aktuellen Version der PH. EUR., Kapitel 2.2.3: pH-Wert - Potentiometrische Methode bestimmt. Das erfindungsgemäße Initiatorsystem ermöglicht die Aushärtung von sauren Zahnharzsystemen in einer anwendungsfreundlichen Abbindezeit, die im Bereich von 0,5 bis 15 min, vorzugsweise im Bereich von 1 bis 10 min liegt, gemessen bei 37°C gemäß ISO 4049:2000.

Die Abbindezeit im Sinne der Erfindung beginnt mit Mischen der Komponenten. An die Abbindezeit schließt sich die Bearbeitungszeit an, in der die Formulierung soweit ausgehärtet ist, dass sie bearbeitet, beispielsweise poliert werden kann.

Das Initiatorsystem funktioniert auf trockenem bis nassem Untergrund und bewirkt eine hohe Haftung des polymerisierten Materials an der Zahnhartsubstanz.

Das erfindungsgemäße Polymerisationsinitiatorsystem kann zur Herstellung und Anwendung von haftvermittelnden Stoffen, dentalen Befestigungsmassen und Zahnfüllmaterialien mit variablem Füllstoffgehalt verwendet werden. Das erfindungsgemäße Polymerisationsinitiatorsystem eignet sich auch zum Härten von Lacken und/oder Klebstoffen auf Basis ethylenisch ungesättigter Monomere.

Das erfindungsgemäße Polymerisationsinitiatorsystem eignet sich insbesondere zum Härten von sauren Formulierungen auf Basis ethylenisch ungesättigter Monomere, beispielsweise (Meth)acrylate.

Die Erfindung wird im folgenden anhand von Beispielen näher erläutert, ohne dass sie durch diese beschränkt werden soll.

### Beispiel 1: Redoxhärtendes 2 K-System (3 Teile Pulver / 1 Teil Flüssigkeit)

Pulver:
88,6 Gew.-% Strontiumaluminiumfluorosilikatglas, silanisiert mit 0,3 Gew.% Methacryloxypropyltrimethoxysilan
1,6 Gew.-% Calciumhydroxid
0,8 Gew.-% Natriumtoluolsulfinat
1,2 Gew.-% 1,3-Dimethyl-5-phenylbarbitursäure
2,4 Gew.-% Natriumperoxodisulfat
5,4 Gew.-% pyrogene Kieselsäure (Aerosil OX 50) silanisiert mit 3 Gew.-% Methacryloxypropyltrimethoxysilan
Flüssigkeit:
49,9 Gew.-% 1,3-Glycerindimethacrylatphosphat
20 Gew.-% propoxyliertes Bisphenol A-dimethacrylat
30 Gew.-% Triethylenglykoldimethacrylat
0,1 Gew.-% Cu(II)-Acetat

### Beispiel 2: Redoxhärtendes 2 K-System (3 Teile Pulver / 1 Teil Flüssigkeit)

Pulver:
93,0 Gew.-% Strontiumaluminiumfluorosilikatglas, silanisiert mit 0,3 Gew.% Methacryloxypropyltrimethoxysilan
1,6 Gew.-% Calciumhydroxid
0,8 Gew.-% Natriumtoluolsulfinat
1,2 Gew.-% 1,3-Dimethyl-5-phenylthiobarbitursäure
2,4 Gew.-% Natriumperoxodisulfat
1,0 Gew.-% pyrogene Kieselsäure (Aerosil OX 50), silanisiert mit 3 Gew.-% Methacryloxypropyltrimethoxysilan

Flüssigkeit:
41,9 Gew.-% Hydroxyethylmethacrylat-phosphat
40 Gew.-% propoxyliertes Bisphenol A-dimethacrylat
18 Gew.-% Triethylenglykoldimethacrylat
0,1 Gew.-% Cu(II)-Acetat

### Vergleichsbeispiel 1: Wie Beispiel 1, aber ohne Natriumperoxodisulfat

Pulver:
91,0 Gew.-% Strontiumaluminiumfluorosilikatglas silanisiert mit 0,3 Gew.% Methacryloxypropyltrimethoxysilan
1,6 Gew.-% Calciumhydroxid
0,8 Gew.-% Natriumtoluolsulfinat
1,2 Gew.-% 1,3-Dimethyl-5-phenylbarbitursäure
5,4 Gew.-% pyrogene Kieselsäure (Aerosil OX 50) silanisiert mit 3 Gew.-% Methacryloxypropyltrimethoxysilan

Flüssigkeit:
49,9 Gew.-% 1,3-Glycerindimethacrylatphosphat
20 Gew.-% propoxyliertes Bisphenol A-dimethacrylat
30 Gew.-% Triethylenglykoldimethacrylat
0,1 Gew.-% Cu(II)-Acetat

### Vergleichsbeispiel 2: Wie Beispiel 1, aber ohne 1,3-Dimethyl-5-phenylbarbitursäure

Pulver:
89,8 Gew.-% Strontiumaluminiumfluorosilikatglas silanisiert mit 0,3 Gew.% Methacryloxypropyltrimethoxysilan
1,6 Gew.-% Calciumhydroxid
0,8 Gew.-% Natriumtoluolsulfinat
2,4 Gew.-% Natriumperoxodisulfat
5,4 Gew.-% pyrogene Kieselsäure (Aerosil OX 50) silanisiert mit 3 Gew.-% Methacryloxypropyltrimethoxysilan

Flüssigkeit:
49,9 Gew.-% 1,3-Glycerindimethacrylatphosphat
20 Gew.-% propoxyliertes Bisphenol A-dimethacrylat
30 Gew.-% Triethylenglykoldimethacrylat
0,1 Gew.-% Cu(II)-Acetat

### Vergleichsbeispiel 3: Wie Beispiel 1, aber ohne Natriumtoluolsulfinat

Pulver:
89,4 Gew.-% Strontiumaluminiumfluorosilikatglas silanisiert mit 0,3 Gew.% Methacryloxypropyltrimethoxysilan
1,6 Gew.-% Calciumhydroxid
1,2 Gew.-% 1,3-Dimethyl-5-phenylbarbitursäure
2,4 Gew.-% Natriumperoxodisulfat
5,4 Gew.-% pyrogene Kieselsäure (Aerosil OX 50) silanisiert mit 3 Gew.-% Methacryloxypropyltrimethoxysilan

Flüssigkeit:
49,9 Gew.-% 1,3-Glycerindimethacrylatphosphat
20 Gew.-% propoxyliertes Bisphenol A-dimethacrylat
30 Gew.-% Triethylenglykoldimethacrylat
0,1 Gew.-% Cu(II)-Acetat

### Vergleichsbeispiel 4: Wie Beispiel 1, aber ohne Cu(II)-Acetat

Pulver:
88,6 Gew.-% Strontiumaluminiumfluorosilikatglas silanisiert mit 0,3 Gew.% Methacryloxypropyltrimethoxysilan
1,6 Gew.-% Calciumhydroxid
0,8 Gew.-% Natriumtoluolsulfinat
1,2 Gew.-% 1,3-Dimethyl-5-phenylbarbitursäure
2,4 Gew.-% Natriumperoxodisulfat
5,4 Gew.-% pyrogene Kieselsäure (Aerosil OX 50) silanisiert mit 3 Gew.-% Methacryloxypropyltrimethoxysilan

Flüssigkeit:
50 Gew.-% 1,3-Glycerindimethacrylatphosphat
20 Gew.-% propoxyliertes Bisphenol A-dimethacrylat
30 Gew.-% Triethylenglykoldimethacrylat

### Beschreibung der durchgeführten Messungen

### Bestimmung der Haftung:

Zur Durchführung von Haftversuchen werden Rinderzähne verwendet. Pro Versuch werden fünf nach dem Extrahieren tiefgefrorene Rinderzähne aufgetaut, von restlichem Zahnfleisch gereinigt und die Wurzel durch Absägen mit einer Diamantsäge abgetrennt. Die noch verbleibende Pulpa wird mit Hilfe einer Pulpanadel entfernt und die Zähne dann mit Leitungswasser gespült. Planes Dentin bzw. Schmelz wird durch labiales Schleifen der Zähne an einer wassergekühlten Diamantschleifscheibe erhalten. Die Zähne werden dann so in Silikon eingebettet, dass die abgeschliffene, gut feucht gehaltene Oberfläche nach oben zeigt und anschließend mit einem feinen Siliziumcarbidschleifpapier nass nachbearbeitet. Dann wird auf jeden Zahn ein Wachsplättchen aufgeklebt, welches eine runde Ausstanzung von 6 mm Durchmesser hat (Prüffeld). Dieses Prüffeld wird nach relativer Trockenlegung mit dem zu testenden Material plan gefüllt und 1 h bei 36°C und 100% rel. Feuchte gehärtet. Nach der Aushärtung wird das Wachsplättchen entfernt, eine Schraube im rechten Winkel zur Zahnoberfläche auf das zu testende Material aufgeklebt und nach einer Lagerung von einem Tag bei 36°C und 100% rel. Feuchte, im Abzugsversuch die Haftung an einer Zwick UPM mit einer Abzugsgeschwindigkeit von 1 mm/min gemessen.

### Bestimmung der Biegefestigkeit

### (3-Punktbiegeversuch nach EN ISO 4049:2000)

Die Ergebnisse der Biegefestigkeits- und Haftmessungen sind in der Tabelle 1 zusammengestellt.

**Tabelle 1**

| Material | Biegefestigkeit [MPa] | Haftung Dentin [MPa] | Haftung Schmelz [MPa] |
|---|---|---|---|
| Beispiel 1 | 58 | 4,1 | 4,4 |
| Beispiel 2 | 54 | 4,7 | 5,1 |
| Vergleichsbeispiel 1 | 56 | 0,0 | 0,0 |
| Vergleichsbeispiel 2 | 9 | 0,0 | 0,0 |
| Vergleichsbeispiel 3 | nicht messbar | nicht messbar | nicht messbar |
| Vergleichsbeispiel 4 | nicht messbar | nicht messbar | nicht messbar |

Vergleichsbeispiel 1 (ohne Natriumperoxodisulfat) zeigt im Gegensatz zu den erfindungsgemäßen Beispielen 1 und 2 keine Haftung auf der Zahnhartsubstanz. Ein Weglassen von anderen Initiatorbestandteilen wie des Barbitursäure(derivats) oder der Sulfinat- oder Kupferverbindung hat zur Folge, dass keine Haftung erzielt wird und/oder dass das Material unvollständig oder gar nicht aushärtet.

### Haftung auf trockenem und nassem Dentin:

Beschreibung der durchgeführten Messungen:
Die Messungen erfolgten an dem 2K-System gemäß Beispiel 1 wie unter "Bestimmung der Haftung" beschrieben. Anstelle der relativen Trockenlegung wurde jedoch in einem Fall das Dentin unter einem Luftstrom vollständig trocken geblasen, im anderen Fall wurde ein Wasserfilm auf dem Dentin belassen. Die Ergebnisse sind der Tabelle 2 zu entnehmen.

**Tabelle 2**

| Oberfläche | Haftung [MPa] |
|---|---|
| Relativ trockengelegtes Dentin | 4,1 |
| Getrocknetes Dentin | 4,0 |
| Wasserfilm auf dem Dentin | 3,6 |

Die in Tabelle 2 dargestellten Ergebnisse zeigen, dass das erfindungsgemäße System sehr tolerant gegenüber der Zahnvorbehandlung ist, da auch in Extremfällen sehr gute Haftungen erzielt werden.

## Patentansprüche

1. Redox-Initiatorsystem, welches folgende Bestandteile umfasst:
(A) 14,9 bis 50 Gew.-% einer Barbitursäure oder Thiobarbitursäure bzw. eines Barbitursäure- oder Thiobarbitursäurederivats,
(B) 30 bis 75 Gew.-% einer Peroxodisulfatverbindung und/oder Peroxodiphosphatverbindung
(C) 10 bis 35 Gew.-% einer Sulfinsäureverbindung und
(D)0,1 bis 5 Gew.-% einer Kupferverbindung.

2. Redox-Initiatorsystem nach Anspruch 1, wobei die Komponente (A) die Struktur aufweist: 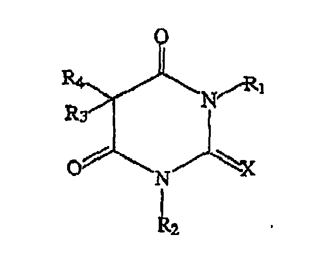 , wobei
X =O oder S;
R₁, R₂, R₃ und R₄, die gleich oder verschieden sein können Wasserstoff, Alkyl-, substituierter Alkyl-, Alkenyl-, Cycloalkyl-, substituierter Cycloalkyl-, Arylalkyl-, Aryl- oder substituierter Arylrest, einen Halogenrest wie Chlor oder eine Hydroxy-. Amino- oder Nitrogruppe bedeuten.

3. Redox-Initiatorsystem nach einem der vorstehenden Ansprüche, wobei Komponente (A) gewählt ist aus: Barbitursäure, Thiobarbitursäure, 1,3,5-Trimethylbarbitursäure, 1-Phenyl-5-benzyl-barbitursäure. 1-Benzyl-5-phenyl-barbitursäure, 1,3-Dimethylbarbitursäure, 1,3-Dimethyl-5-phenyl-barbitursäure, 1-Gyclohexyl-5-ethyl-barbitursäure, 5-Laurylbarbitursäure. 5-Butylbarbitursäure, 5-Allylbarbitursäure, 5-Hydroxy-5-butylbarbitursäure, 5-Phenylthiobarbitursäure, 1,3-Dimethylthiobarbitursäure, 5.5-Dibrombarbitursäure. Trichlorbarbltursäure, 5-Nitrobarbitursäure, 5-Aminobarbitursäure. 5-Hydroxybarbitursäure und 5,5-Dihydroxybarbitursäure.

4. Redox-Initiatorsystem nach einem der vorstehenden Ansprüche, wobei die Komponente (B) eine Wasserlöslichkeit von größer 4 g / 100 ml Wasser bei 20°C aufweist.

5. Redox-Initiatorsystem nach einem der vorstehenden Ansprüche, wobei die Komponente (B) gewählt ist aus:
Ammonium-, Natrium- und Kaliumperoxodisutfatverbindungen bzw.- peroxodiphosphatverbindungen.

6. Redox-Initiatorsystem nach einem der vorstehenden Ansprüche, wobei es sich bei der Komponente (C) um eine Sulfinsäureverbindung der allgemeinen Formel R₁SOO-R₂ handelt, mit R₁ = Alkyl-, substituierter Alkyl-, Alkonyl-, Cycloalkyl-, substituierter Cycloalkyl-, Arylalkyl-, Aryl- oder substituierter Arylrest ist und R₂ = H, Metall oder Alkyl-, substituierter Alkyl-, Alkenyl-, Cycloalkyl-, substituierter Cycloalkyl-, Arylalkyl-, Aryl- oder substituierter Arylrest.

7. Redox-Initiatorsystem nach Anspruch 5, wobei die Komponente (C) gewählt ist aus: Natriumbenzolsulfinat und/oder Natriumtoluolsulfinat bzw. deren Hydrate, Benzolsulfinsäure, Natriumbenzolsulfinatdihydrat, Formamidinsuffinsäure, Natriumsalz der Hydroxymethansulfinsäure, Natriumsalz der 2,5-Dichlorbenzolsulfinsäure und 3-Acetamido-4-methoxybenzolsulfinsäure.

8. Redox-Initiatorsystem nach einem der vorstehenden Ansprüche, wobei die Komponente (D) gewählt- ist aus: Kupterchlorid, Kupferacetat, Kupferacetylacetonat, Kupfernaphthenat, Kupfersalicylat oder Komplexe von Kupfer mit Thioharnstoff oder Ethylendiamintetraessigsäure.

9. Redox-Initiatorsystem nach einem der vorstehenden Ansprüche als Bestandteil einer radikalisch polymerisierbaren Masse mit pH-Werten kleiner 5 nach dem Mischen mit dem Redox-Initiatorsystem.

10. Redox-Initiatorsystem nach Anspruch 8, wobei die Masse säurefunktionelle (Meth)acrylate umfasst.

11. Redox-Initiatorsystem nach einem der vorstehenden Ansprüche als Bestandteil von Dentalformulierungen, Klebstoffen oder Lacken.

12. Verwendung eines Redox-Initiatorsystem nach einem der vorstehenden Ansprüche 1 bis 7 zum Härten von radikalisch polymerisierbaren, sauren Formulierungen.

## Claims

1. Redox initiator system which comprises the following constituents:
(A) from 14.9 to 50% by weight of a barbituric acid or thiobarbituric acid and/or of a barbituric or thiobarbituric acid derivative,
(B) from 30 to 75% by weight of a peroxodisulfate compound and/or peroxodiphosphate compound,
(C) from 10 to 35% by weight of a sulfinic acid compound, and
(D) from 0.1 to 5% by weight of a copper compound.

2. Redox initiator system according to claim 1, wherein component (A) has the following structure: 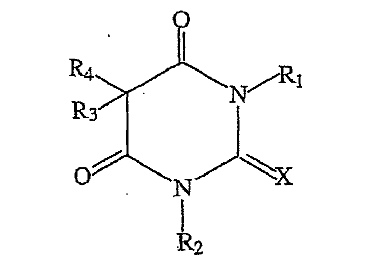 where X = 0 or S;
R₁, R₂, R₃, and R₄, which may be identical or different, are hydrogen, alkyl, substituted alkyl, alkenyl, cycloalkyl, substituted cycloalkyl, arylalkyl, aryl or substituted aryl radical, a halogen radical such as chloro or a hydroxyl, amino or nitro group.

3. Redox initiator system according to one of the above claims, wherein component (A) is selected from barbituric acid, thiobarbituric acid, 1,3,5-trimethylbarbituric acid, 1-phenyl-5-benzylbarbituric acid, 1-benzyl-5-phenylbarbituric acid, 1,3-dimethylbarbituric acid, 1,3-dimethyl-5-phenylbarbituric acid, 1-cyclohexyl-5-ethylbarbituric acid, 5-laurylbarbituric acid, 5-butylbarbituric acid, 5-allylbarbituric acid, 5-hydroxy-5-butylbarbituric acid, 5-phenylthiobarbituric acid, 1,3-dimethylthiobarbituric acid, 5,5-dibromobarbituric acid, trichlorobarbituric acid, 5-nitrobarbituric acid, 5-aminobarbituric acid, 5-hydroxybarbituric acid and 5,5-dihydroxybarbituric acid.

4. Redox initiator system according to one of the above claims, wherein component (B) has a water-solubility of more than 4 g/100 ml water at 20°C.

5. Redox initiator system according to one of the above claims, wherein component (B) is selected from ammonium-, sodium- and potassiumperoxodisulfate compounds or peroxodiphosphate compounds.

6. Redox initiator system according to one of the above claims, wherein component (C) comprises a sulfinic acid compound of the general formula R₁SOO-R₂, with R₁ = alkyl, substituted alkyl, alkenyl, cycloalkyl, substituted cycloalkyl, arylalkyl, aryl or substituted aryl radical and R₂ = H, metal or alkyl, substituted alkyl, alkenyl, cycloalkyl, substituted cycloalkyl, arylalkyl, aryl or substituted aryl radical.

7. Redox initiator system according to claim 5, wherein component (C) is selected from sodium benzenesulfinate and/or sodium toluenesulfinate and their hydrates, benzenesulfinic acid, sodium benzenesulfinate dihydrate, formamidinesulfinic acid, sodium salt of hydroxymethanesulfinic acid, sodium salt of 2,5-dichlorobenzenesulfinic acid and 3-acetamido-4-methoxybenzenesulfinic acid.

8. Redox initiator system according to one of the above claims, wherein component (D) is selected from copper chloride, copper acetate, copper acetylacetonate, copper naphtheneate, copper salicylate or complexes of copper with thiourea or ethylenediaminetetraacetic acid.

9. Redox initiator system according to one of the above claims as a constituent of a free-radically polymerizable composition having pH values of less than 5 after mixing with the redox initiator system.

10. Redox initiator system according to claim 8, wherein the composition comprises acid-functional (meth)acrylates.

11. Redox initiator system according to one of the above claims as a constituent of dental formulations, adhesives or coating materials.

12. Use of a redox initiator system according to one of the above claims 1 to 7 for curing free-radically polymerizable, acidic formulations.

## Revendications

1. Système d'initiateur redox, comportant les constituants suivants :
(A) 14,9 à 50 % en poids d'un oxyde barbiturique ou d'un acide thiobarbiturique ou bien d'un dérivé d'acide barbiturique ou d'un dérivé d'acide thiobarbiturique,
(B) 30 à 75 % en poids d'un composé de peroxodisulfate et/ou d'un composé de peroxodiphosphate
(C) 10 à 35 % en poids d'un composé d'acide sulfinique et
(D) 0,1 à 5 % en poids d'un composé de cuivre.

2. Système d'initiateur redox selon la revendication 1, dans lequel le composant (A) présente la structure : 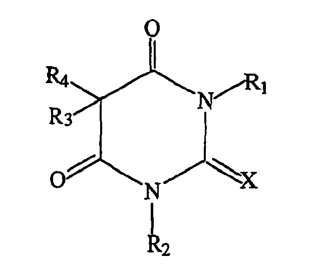 dans laquelle :
X = O ou S ;
R₁, R₂, R₃ et R₄, qui peuvent être identiques ou différents, signifient hydrogène, radical alkyle, radical alkyle substitué, radical alcényle, radical cycloalkyle, radical cycloalkyle substitué, radical arylalkyle, radical aryle ou radical aryle substitué, un radical halogène comme du chlore ou un groupe hydroxy, un groupe amino ou un groupe nitro.

3. Système d'initiateur redox selon l'une quelconque des revendications précédentes, dans lequel le composant (A) est choisi parmi : acide barbiturique, acide thiobarbiturique, acide 1,3,5-triméthylbarbiturique, acide 1-phényl-5-benzylbarbiturique, acide 1-benzyl-5-phénylbarbiturique, acide 1,3-diméthylbarbiturique, acide 1,3-diméthyl-5-phénylbarbiturique, acide 1-cyclohexyl-5-éthylbarbiturique, acide 5-laurylbarbiturique, acide 5-butylbarbiturique, acide 5-allylbarbiturique, acide 5-hydroxy-5-butylbarbiturique, acide 5-phénylthiobarbiturique, acide 1,3-diméthylthiobarbiturique, acide 5,5-dibromobarbiturique, acide trichlorobarbiturique, acide 5-nitrobarbiturique, acide 5-aminobarbiturique, acide 5-hydroxybarbiturique et acide 5,5-dihydroxybarbiturique.

4. Système d'initiateur redox selon l'une quelconque des revendications précédentes, dans lequel le composant (B) présente une solubilité dans l'eau supérieure à 4 g/100 ml d'eau à 20 °C.

5. Système d'initiateur redox selon l'une quelconque des revendications précédentes, dans lequel le composant (B) est choisi parmi : composés de peroxodisulfate d'ammonium, de peroxodisulfate de sodium et de peroxodisulfate de potassium ou composés de peroxodiphosphate d'ammonium, de peroxodiphosphate de sodium et de peroxodiphosphate de potassium.

6. Système d'initiateur redox selon l'une quelconque des revendications précédentes, dans lequel il s'agit, en ce qui concerne le composant (C), d'un composé d'acide sulfinique de la formule générale R₁SOO-R₂, avec R₁ = radical alkyle, radical alkyle substitué, radical alcényle, radical cycloalkyle, radical cycloalkyle substitué, radical arylalkyle, radical aryle ou radical aryle substitué et R₂ = H, métal ou radical alkyle, radical alkyle substitué, radical alcényle, radical cycloalkyle, radical cycloalkyle substitué, radical arylalkyle, radical aryle ou radical aryle substitué.

7. Système d'initiateur redox selon la revendication 5, dans lequel le composant (C) est choisi parmi : benzènesulfinate de sodium et/ou toluènesulfinate de sodium ou des hydrates de ceux-ci, acide benzènesulfinique, dihydrate de benzènesulfinate de sodium, acide formamidinesulfinique, sel de sodium de l'acide hydroxyméthanesulfinique, sel de sodium de l'acide 2,5-dichlorobenzènesulfinique et de l'acide 3-acétamido-4-méthoxybenzènesulfinique.

8. Système d'initiateur redox selon l'une quelconque des revendications précédentes, dans lequel le composant (D) est choisi parmi : chlorure de cuivre, acétate de cuivre, acétylacétonate de cuivre, naphténate de cuivre, salicylate de cuivre ou des complexes de cuivre avec de la thiourée ou de l'acide éthylènediaminetétraacétique.

9. Système d'initiateur redox selon l'une quelconque des revendications précédentes en tant que constituant d'une masse pouvant être polymérisée par voie radicalaire ayant des valeurs de pH inférieures à 5 après le mélange avec le système d'initiateur redox.

10. Système d'initiateur redox selon la revendication 8, dans lequel la masse comporte des (méth)acrylates à fonctionnalité acide.

11. Système d'initiateur redox selon l'une quelconque des revendications précédentes en tant que constituant de formulations dentaires, de colles ou de vernis.

12. Utilisation d'un système d'initiateur redox selon l'une quelconque des revendications précédentes 1 à 7 pour le durcissement de formulations acides, pouvant être polymérisées par voie radicalaire.
